(19) Europäisches Patentamt
European Patent Office
Office européen des brevets

(11) Veröffentlichungsnummer: **0 533 892 B1**

(12) # EUROPÄISCHE PATENTSCHRIFT

(45) Veröffentlichungstag der Patentschrift: **14.06.95**

(51) Int. Cl.$^{6}$: **C07C 47/347**, C11B 9/00, A61K 7/46

(21) Anmeldenummer: **92908834.2**

(22) Anmeldetag: **10.04.92**

(86) Internationale Anmeldenummer: **PCT/DE92/00307**

(87) Internationale Veröffentlichungsnummer: **WO 92/18451 (29.10.92 92/27)**

(54) **8-EXO-FORMYL-2,6-EXO-TRICYCLO[5.2.1.0/2,6]DECAN, VERFAHREN ZU SEINER HERSTELLUNG SOWIE VERWENDUNG DESSELBEN.**

(30) Priorität: **12.04.91 DE 4112093**

(43) Veröffentlichungstag der Anmeldung: **31.03.93 Patentblatt 93/13**

(45) Bekanntmachung des Hinweises auf die Patenterteilung: **14.06.95 Patentblatt 95/24**

(84) Benannte Vertragsstaaten: **CH DE ES FR GB LI NL**

(56) Entgegenhaltungen:
**WO-A-84/03086**
**US-A- 3 270 061**

(73) Patentinhaber: **Dragoco Gerberding & Co Aktiengesellschaft**
**Dragocostrasse 1**
**D-37603 Holzminden (DE)**

(72) Erfinder: **BRUNKE, Ernst-Joachim**
**Pippingsbusch 3**
**D-3450 Holzminden (DE)**
Erfinder: **KAPPEY, Claus-Hermann**
**Schratweg 1**
**D-3450 Holzminden (DE)**
Erfinder: **HÖLSCHER, Bernd**
**Hamelner Strasse 1**
**D-3451 Halle (DE)**
Erfinder: **STRUWE, Hartmut**
**Am Feldberg 25**
**D-3470 Höxter/Stahle (DE)**

(74) Vertreter: **Eikenberg, Kurt-Rudolf**
**Patentanwalt**
**Schackstrasse 1**
**D-30175 Hannover (DE)**

**EP 0 533 892 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

In der modernen, industriellen Parfümerie besteht ein ständiger Bedarf an neuen Riechstoffen mit möglichst originellen und ästhetischen Geruchsnoten bei zugleich hoher Stabilität und somit vielseitigen technischen Einsatzmöglichkeiten. Ein weiterer wichtiger Gesichtspunkt der industriellen Riechstoffproduktion ist die Bereitstellung derartiger neuer Riechstoffe, ausgehend von synthetischen Rohstoffen, die in großer Menge und preiswert zur Verfügung stehen.

Die vorliegende Erfindung betrifft 8-exo-Formyl-2,6-exo-tricyclo[5.2.1.$0^{2,6}$]decan (**1**), das in reiner, bzw. in einer neben seinem 8-endo-Isomerem **2** angereicherten Form, ein wertvoller neuer Riechstoff ist.

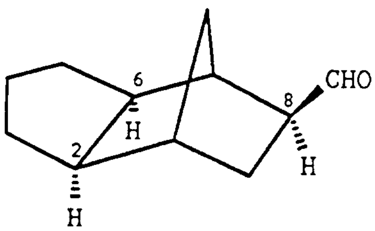

1 (d, l)

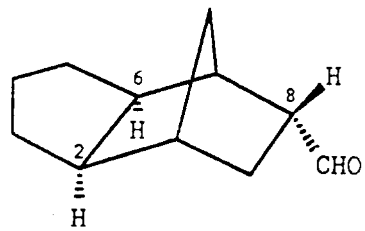

2 (d, l)

Es ist neu, daß der Aldehyd **1** mit der angegebenen Konstitution und Stereochemie über besonders positive geruchliche Eigenschaften verfügt und ferner aufgrund seines Verhaltens in parfümistisch-technischen Anwendungen von besonderem industriellen Wert ist. Die Verbindung **1** mit ihrer hier angegebenen Konstitution und relativen Konfiguration ist bisher nicht beschrieben worden.

C-8-substituierte Tricyclo [5.2.1.$0^{2,6}$]decan-Derivate ohne Angabe der Stereochemie an Position C-8 werden im US-Patent 3,270,061 (August 30, 1966) beschrieben. Hierunter befindet sich auch die 8-Formyl-Verbindung **D**, die aber nicht in die Ansprüche des genannten Patents einbezogen worden ist.

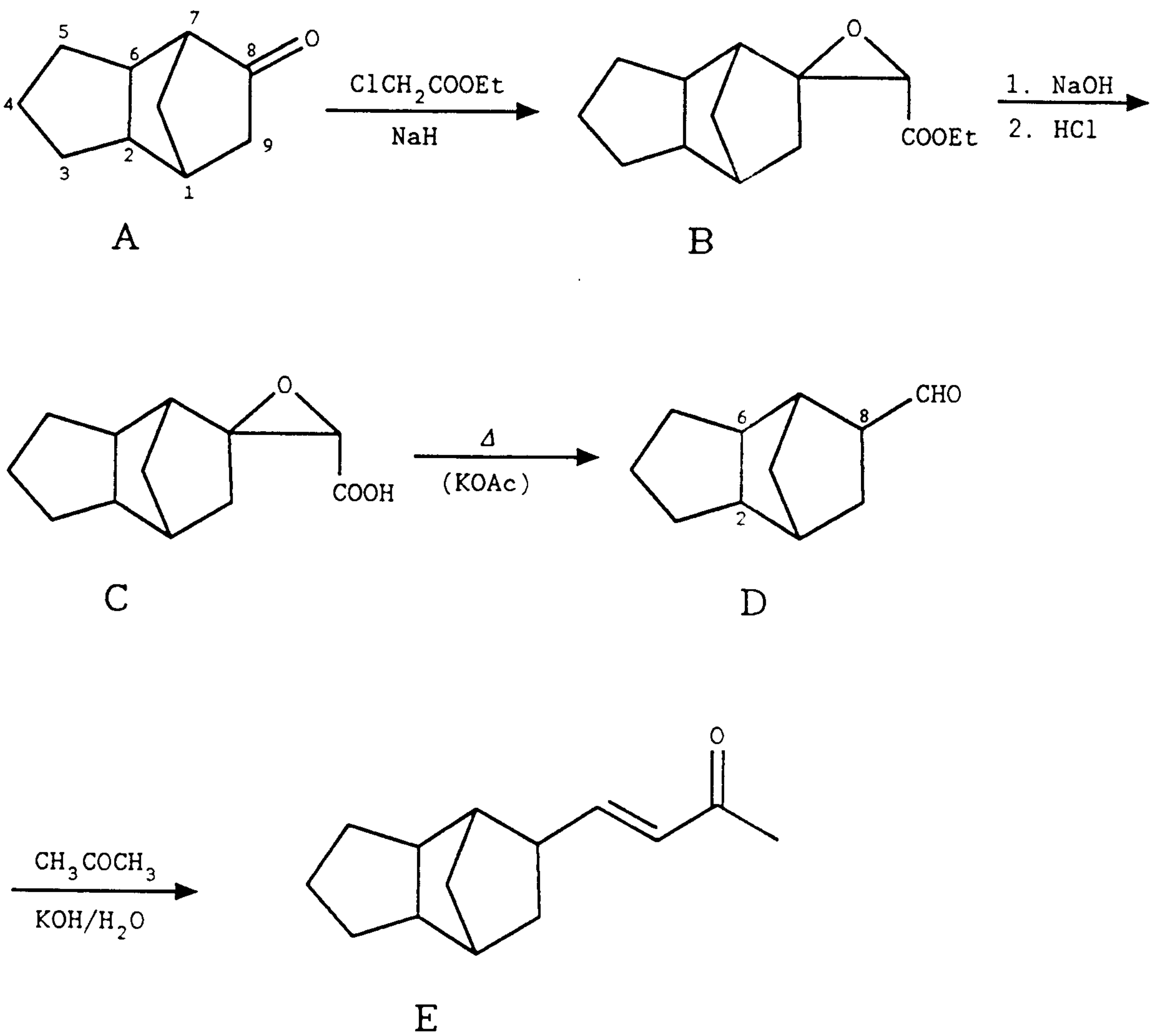


A B C D E

In dieser Schrift wird die Stereochemie des Ringsystems als 2,6-exo beschrieben, aber nicht als bedeutungsvoll für Geruchseigenschaften der genannten bzw. beanspruchten Verbindungen angesehen. So wird in dem genannten Patent in Spalte 1 in den Zeilen 69 bis 72 ausgesagt, daß die beschriebenen Verbindungen üblicherweise und bevorzugt aus den 2,6-exo-Isomeren bestehen, daß jedoch die korrespondierenden endo-Isomeren in gleicher Weise im Sinn der vorliegenden Erfindung genutzt werden könnten. Über die mögliche Bedeutung der Stereochemie an C-8 wurde ebenfalls nichts gesagt. Es ist daher neu und überraschend, daß mit dem Aldehyd **1**, d. h. bei ausschließlichem Vorliegen der 2,6-exo-Konfiguration und gleichzeitiger exo-Konfiguration der Formylgruppe an C-8 ein Riechstoff vorliegt, der sich durch eine besonders natürliche und ästhetisch wertvolle Grünnote auszeichnet.

Für den gemäß US-Patent 3,270,061 erhaltenen Aldehyd **D** gleicher Konstitution wird dort ein intensiver grüner Geruch mit erdigen Obertönen angegeben (Spalte 5, Zeilen 69 bis 71). Es wurde in der genannten Schrift ferner ausgesagt, daß der Aldehyd **D** von Wert für die Formulierung schwererer Blumennoten z. B. Veilchennoten geeignet sei. Dennoch wurde der Aldehyd **D** nicht in dem genannten US-Patent als Riechstoff beansprucht, sondern vielmehr nur die aus ihm durch Folgereaktionen hergestellten Ketone **E**, deren Riechstoffeigenschaften ausführlich beschrieben worden sind. Man kann davon ausgehen, daß der Aldehyd **D** aufgrund seiner erdigen Nebennote von begrenztem Wert war und daher keinen Eingang in die internationale Parfümerie gefunden hat.

Der Aldehyd **D** kann analog dem US-Patent 3,270,061 aus dem tricyclischen Keton **A** (TCD-Keton A, kommerzielles Produkt der Fa. Hoechst, Deutschland) hergestellt werden: Durch Umsetzung mit Chloressigsäureethylester in Gegenwart von Natriumhydrid wurde der Glycidester **B** erhalten; die Verseifung von **B** ergab die Glycidsäure **C**, die wiederum durch Erhitzen mit Kaliumacetat in den Aldehyd **D** überführt wurde. Bei Nacharbeitung der im US- Patent Nr. 3,270,061 gegebenen Vorschrift wurde der Aldehyd **D** von uns in

einer Reinheit von ca. 99 % erhalten (Beispiel 1). Die bekannten Geruchseigenschaften konnten von uns bestätigt werden. Wir haben nun überraschenderweise festgestellt, daß unter Einwirkung von Säuren oder Basen, vorzugsweise Alkalihydroxiden oder Alkalicarbonaten in alkoholischer Lösung eine deutliche geruchliche Verbesserung des Aldehyds **D** festzustellen ist. Der Geruch des behandelten Materials ist nicht nur deutlich stärker, sondern auch mehr natürlich-grün.

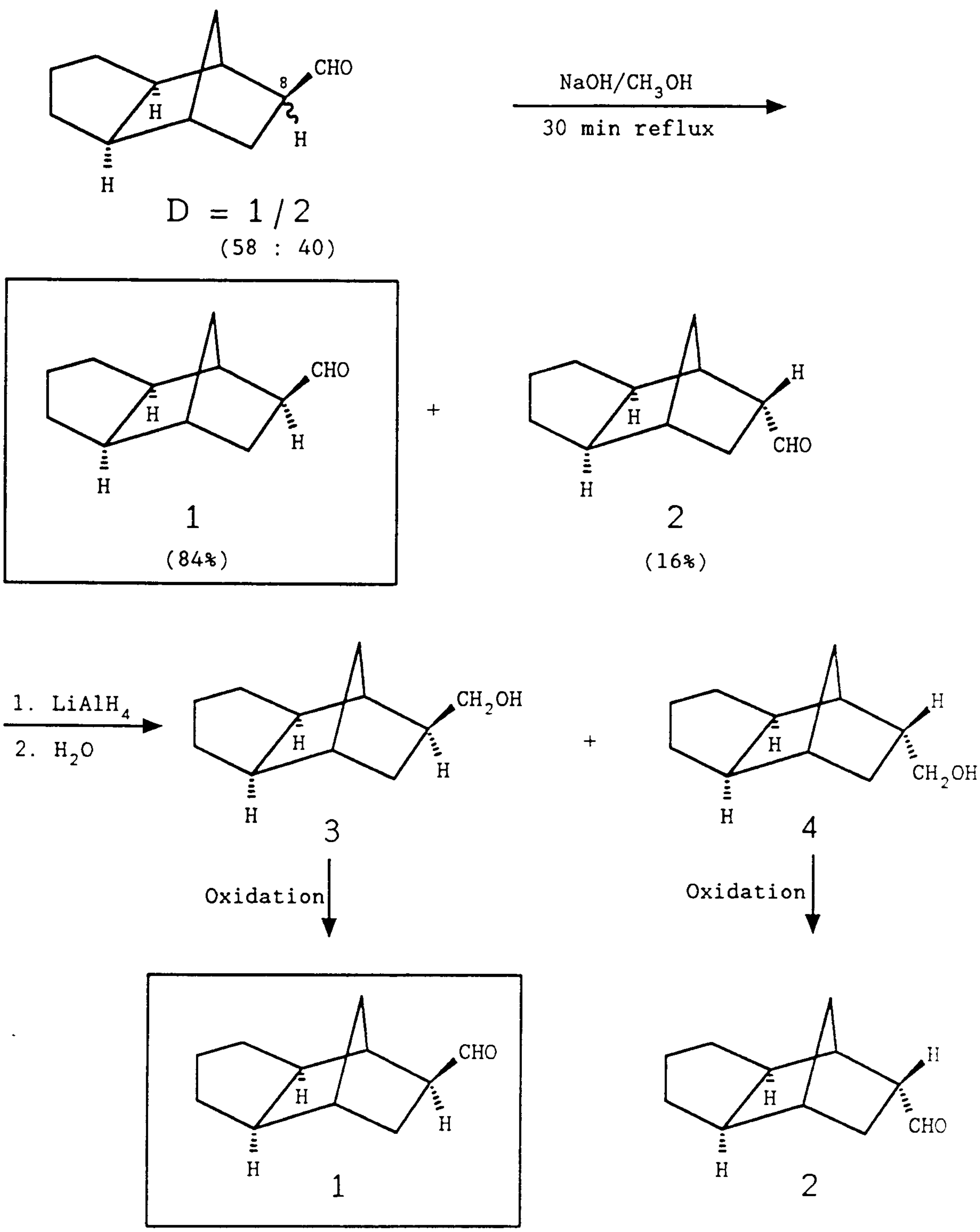


D = 1 / 2
(58 : 40)

1
(84%)

2
(16%)

3

4

1

2

Die analytische Prüfung des analog US Patent 3,270,061 dargestellten Aldehyds **D** auf das Vorliegen anderer Verbindungen nach Einwirkung von Säuren und Basen ist auch mit den modernen analytischen Methoden schwierig. Das gemäß US-Patent 3,270,061 hergestellte Material (Beispiel 1) war in unseren

Händen gaschromatographisch einheitlich. Nach Baseneinwirkung (Beispiel 3a) ist gaschromatographisch auch an hochwirksamen Quarzkapillarsäulen keine Peakauftrennung zu beobachten; dementsprechend wäre das Material unverändert. Wir stellten zur Aufklärung der beobachteten olfaktorischen Unterschiede aus dem vermutlich im Gemisch vorliegenden Aldehyd das korrespondierende Alkoholgemisch in an sich bekannter Weise durch Reduktion mit Lithiumaluminiumhydrid her. Hier können wahlweise andere Reduktionsmittel wie Natriumborhydrid oder auch katalytische Verfahren zum Einsatz kommen. Es wurde überraschenderweise gefunden, daß sich durch Reduktion ein Alkoholgemisch bildet hatte, das sich chromatographisch trennen ließ. Dies zeigte sich u. a. in deutlich unterschiedlichen gaschromatographischen Retentionszeiten und signifikant unterschiedlichen Massenspektren. Durch destillative oder chromatographische Trennung haben wir die Alkohole **3** und **4** isoliert und spektroskopisch charakterisiert.

Durch Reduktion des stereoisomeren Aldehydgemischs zum Alkoholgemisch, dessen Auftrennung durch physikalische Methoden und anschließende Oxidation, sind 8-exo-Formyl-2,6-exo-tricyclo-[5.2.1.$0^{2,6}$]-decan (**1**) und sein 8-endo-Formyl-Isomeres in reiner Form zugänglich. Eine direkte chromatographische oder destillative Auftrennung der stereoisomeren Aldehyde **1/2** war nicht praktikabel. Die stereoisomerenreinen Aldehyde **1/2** lassen sich durch Einwirkung von Säuren oder Basen, vorzugsweise methanolischer Natronlauge, equilibrieren. Das exo/endo-Isomerenverhältnis an C-8 liegt im Gleichgewichtsgemisch bei 84:16. Wie von uns durch Reduktion zum Alkoholgemisch und anschließende gaschromatographische Isomerenbestimmung festgestellt, lag das Aldehydgemisch gemäß US-Patent 3,270,061 im exo/endo-Isomerenverhältnis an C-8 bei 58:40. Unter Equilibrierungsbedingungen konnten wir aus diesem Isomerengemisch ebenfalls ein Produkt mit dem 8-exo/endo-Verhältnis von 84:16 erhalten. Die Einwirkung starker Basen, insbesondere von Alkalihydroxiden in alkoholischen Lösungen, erhöht das C-8-exo/endo-Verhältnis von 1,45:1 auf 5,25:1 und kann somit als Verfahren zur Anreicherung des olfaktorisch bevorzugten Stereoisomers genutzt werden.

Die Glycidestersynthese an TCD-Keton A (**A**) kann in Gegenwart von Antioxidantien, z. B. Phenothiazin in Pyridin unter Verwendung von Natriummethylat durchgeführt werden (Beispiel 3b). In einer Eintopf-Reaktion können die Verseifung zur Glycidsäure mit methanolischer Natronlauge und die Decarboxylierung unter Umwandlung in den Aldehyd nach Zugabe

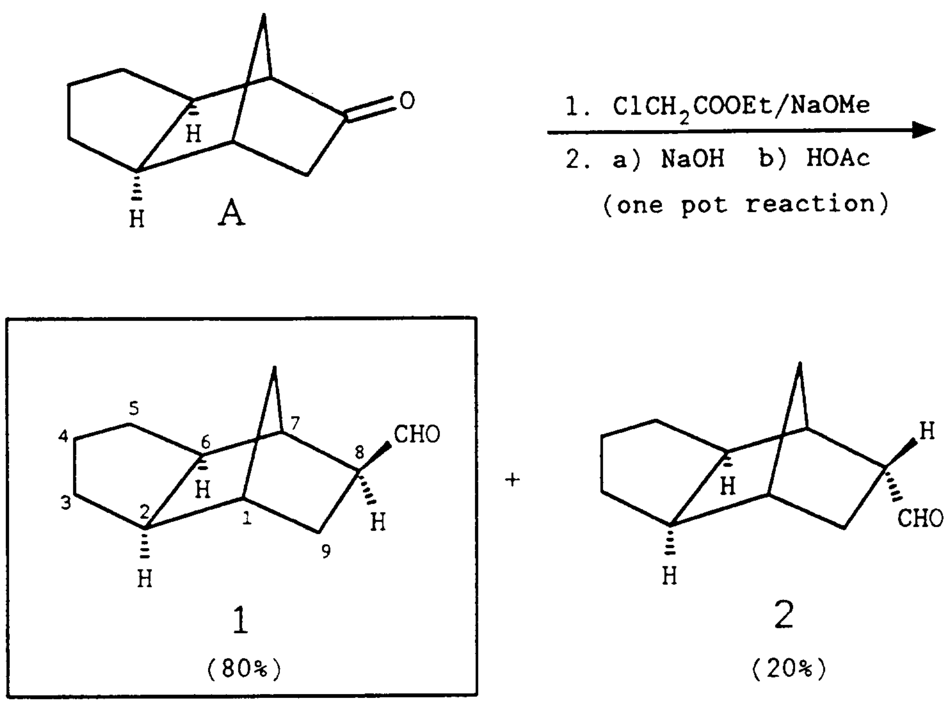


A

1 (80%) + 2 (20%)

von Essigsäure (d.h. in Gegenwart von Natriumacetat) ausgeführt werden. Die Ausführung der Reaktionssequenz als Eintopf-Reaktion führt zu relativ niedrigen Produktionskosten. Die pH-Werteinstellung bei Decarboxylierung und Aufarbeitung bestimmt das Isomerenverhältnis der C-8-Formylgruppe (C-8-exo/endo = 80:20). Das nach diesem Verfahren hergestellte Roh-Aldehydgemisch enthält noch kleine Restmengen des Ausgangsmaterials TCD-Keton A. Das C-8-exo-angereicherte Aldehydgemisch **1/2** (80:20) kann über sein Bisulfitaddukt in hoher Reinheit gewonnen werden.

Eine alternative Synthesesequenz geht aus von 2,6-exo-Dicyclopentadien (**5**), das durch selektive Hydroformylierung an der reaktiveren Doppelbindung im Norbornenteil des 2,6-exo-Dicyclo-pentadiens in das ungesättigte Aldehydgemisch **6** überführt werden kann. Die katalytische Hydrierung von **6** ergibt das Aldehydgemisch **1/2** in einem C-8-exo/endo-Verhältnis von 4,3:1. Derartige regioselektive Hydroformylierungen sind beispielsweise in der DE OS 3447030 beschrieben worden. Die katalytische Hydrierung des ungesättigten Aldehydgemisches **6** erfolgt vorzugsweise unter Verwendung von Palladiumkohle unter kontrollierten Reaktionsbedingungen.

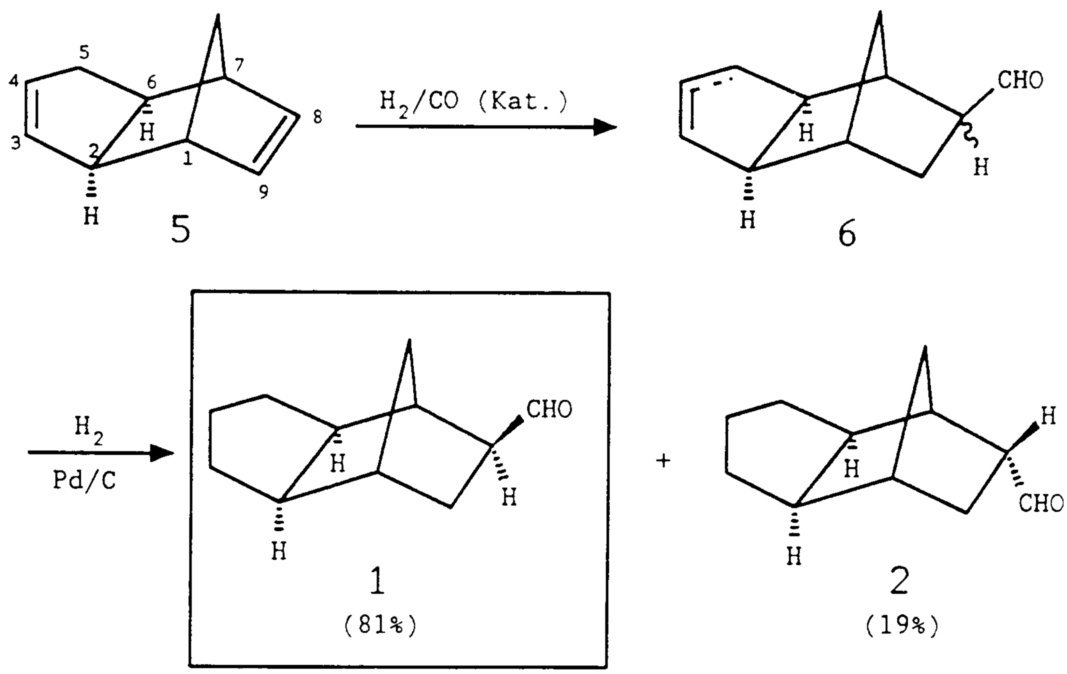


5 6

1 (81%) + 2 (19%)

Ein weiterer alternativer Reaktionsweg geht aus von 2,6-exo-Tricyclo[5.2.1.0.$^{2,6}$]decen-8 (**7**), dessen Hydroformylierung direkt das Aldehydgemisch **1/2** ergibt, das wiederum durch Equilibrierung in das Stereoisomerengemisch **1/2** mit hohem 8-exo-Anteil überführt werden kann.

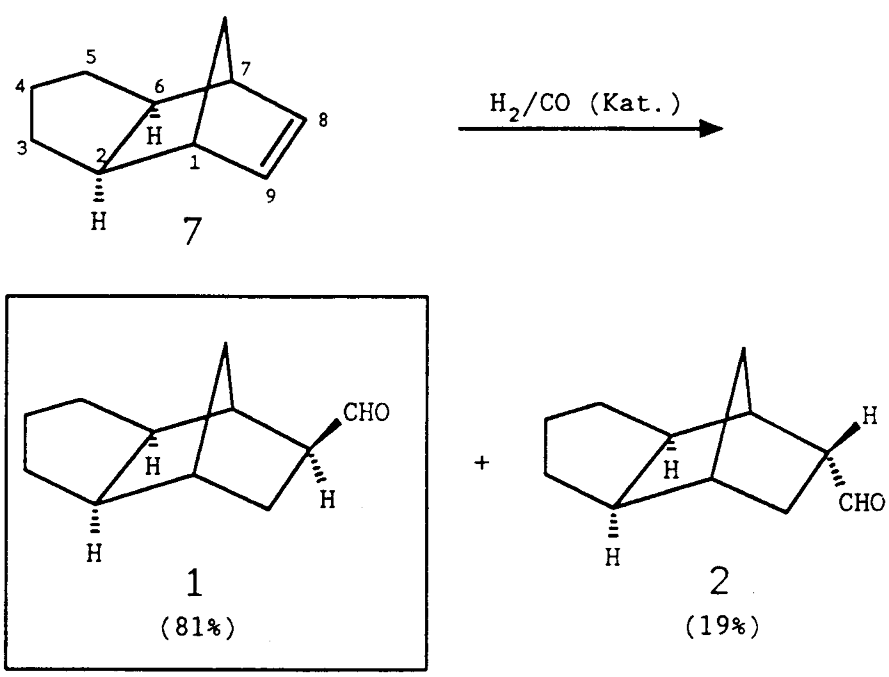


7

1 (81%) + 2 (19%)

Das 8-exo-Formyl-2,6-tricyclodecan (**1**) zeichnet sich gegenüber seinem C-8-Epimeren **2** durch besonders günstige Geruchseigenschaften aus. Der olfaktorische Wert der Verbindung **1** kommt auch in Gemischen der Diastereomeren **1** und **2** zum Ausdruck, wenn **1** angereichert vorliegt, vorzugsweise zumindestens 70 % neben 30 % der Verbindung **2**. Die Geruchsnote der Verbindung **1** kann als frisch-grün bezeichnet werden und die der Verbindung **2** als erdig, moderig, grün. Die Verbindung **1** unterscheidet sich von der Stereoisomerenverbindung **2** nicht nur durch die vorteilhaftere Geruchsnote, sondern auch durch eine deutlich größere Geruchsstärke. Der olfaktorische Wert der Verbindung **1** in reiner bzw. angereicherter Form (**1/2** = 70 : 30) kommt in Parfümkompositionen selbst bei relativ niedriger Dosierung zur vollen Geltung (siehe Beispiele 8, 9, 10). Bereits eine Anreicherung des 8-exo-Isomeren **1** um 10 % gegenüber dem Stand der Technik führte zu einer deutlichen Verbesserung des Geruchseffekts im parfümistischen Akkord gemäß Beispiel 8. Dieser deutliche und positive Effekt verstärkt sich bei weiterer Anreicherung der Verbindung **1**. Die sehr gute Stabilität und Substantivität der Verbindung **1** in reiner oder angereicherter Form und die geschilderten positiven Geruchseigenschaften machen die Verbindung **1** in reiner oder angereicherter Form zu einem neuen und wertvollen Riechstoff.

Beispiel 1

Herstellung von 8-exo/endo-Formyl-2,6-exo-tricyclo [5.2.1.$0^{2,6}$]decan (**D** = **1/2**) gemäß US-PS 3,270,061 a) Spalte 4, Zeilen 26-66; b) Spalte 5, Zeilen 19-65)

a) Darstellung von Glycidester **B** aus Keton **A**

Eine Lösung von 270 g (1.8 mol) TCD-Keton-A (**A**) und 245,2g (2 mol) Chloressigsäureethylester in 900 ml abs. Toluol wird unter Rühren innerhalb von 3 h bei 30 - 35 °C portionsweise mit 60 g (2 mol) 80 %iger Natriumhydrid-Dispersion in Mineralöl versetzt, wobei aufgrund exothermer Reaktion Eiswasserkühlung erforderlich ist. Nach einer weiteren Stunde Rühren bei 30 - 35 °C klang die exotherme Reaktion ab. Anschließend wurde das Reaktionsgemisch ohne weitere Kühlung über Nacht weitergerührt. Danach wurde das überschüssige Natriumhydrid durch vorsichtige Zugabe von 50 ml Methanol zerstört. Das Reaktionsgemisch wurde mit 500 ml Wasser verdünnt und mit 15 ml Eisessig schwach sauer gestellt. Nach Abtrennung der organischen Phase wurde die wäßrige Phase mit 150 ml Toluol extrahiert. Die vereinigten Toluol-Phasen wurden mit $NaHCO_3$-Lösung und Wasser neutralgewaschen und schließlich im 20 mm-Vakuum vom Lösungsmittel befreit. Das verbliebene Rohprodukt (412,9g) wurde über eine 20cm-Vigreux-Kolonne destilliert.

Man erhielt 302,6 g Glycidester **B** im Siedebereich 139-145 °C/1,5 mm. Gaschromatogramm (30 m DBWAX, 100-240 °C, 6 °C/min): $t_R$ = 15.3 (20.4 %), 15.7 (29.5 %), 16.2 (20.3 %), 16.9 min (28.0 %).

b) Darstellung von Aldehyd **D** via Glycidsäure **C**

Eine Lösung von 118 g (0.5 mol) Glycidester **B** in 275 ml Methanol wird unter Rühren innerhalb von 30 min mit einer Lösung von 40 g NaOH (1 mol) in 350 ml $H_2O$ versetzt, wobei aufgrund leicht exothermer Reaktion Kaltwasserkühlung erforderlich ist. Anschließend wurde das Reaktionsgemisch ohne weitere Kühlung 17 h gerührt. Dann wurde mit einer Lösung von 112 g conc. HCl in 250ml $H_2O$ auf pH = 3 angesäuert und 2 mal mit je 150ml Toluol extrahiert. Der Toluol-Extrakt wurde mit Wasser auf pH = 5,5 gewaschen und im 20 mm-Vakuum vom Lösungsmittel befreit. Man erhielt 98,5 g rohe Glycidsäure **C** als Rückstand, der unter Zusatz von 3 g Kaliumacetat im Vakuum erhitzt wird, wobei das gebildete Produkt über eine 20 cm-Vigreux-Kolonne abdestilliert wird. Man erhält 71,5 g Aldehyd **D** (62 % d. Th., bezogen auf TCD-Keton-A (**A**)) im Siedebereich 82 - 87 °C/ 1,5 - 2 mm als farbloses Öl.
Gaschromatogramm (30 m DBWAX, 100 - 240 °C, 6 °C/min): $t_R$ = 13.0 min (98.6 %).
Brechungsindex: $n_D^{20}$ = 1.5020 (Lit.: $n_D^{20}$ = 1.4999)
Dichte $D_4^{20}$ = 1.0376

Beispiel 2

a) Reduktion des Aldehydgemisches **D** (**1/2**) aus Beispiel 1

Zu einer Suspension von 570 mg (15 mmol) $LiAlH_4$ in 10 ml abs. Diethylether wurde bei 0 °C unter Rühren und Kühlen eine Lösung von 8,2 g (50 mmol) Aldehyd **D** aus Beispiel 1 in 20 ml abs. Diethylether getropft. Man ließ 30 min bei Raumtemperatur nachrühren, zerstörte den $LiAlH_4$-Überschuß durch Zugabe

von 1 ml Ethylacetat, hydrolysierte mit 5 ml Eiswasser und filtrierte die entstandene Suspension über Theorit. Das Filtrat wurde im 20 mm-Vakuum vom Lösungsmittel befreit. Man erhielt 8,2 g Alkoholgemisch **3/4** als farbloses Öl.
Gaschromatogramm (30 m DBWAX, 100 - 240 °C, 6 °C/min):
$t_R$ = 17.8 (40 %, endo-Isomer), 18.3 min (58 %, exo-Isomer).

b) Trennung des Alkoholgemisches **3/4**

82 g Alkoholgemisch **3/4** vorstehender Zusammensetzung, das durch Reduktion der entsprechenden Menge Aldehyd **D** aus Beispiel 1 erhalten wurde, wurden einer fraktionierenden Destillation bei 110 - 111 °C und 3,5 mbar an einer 1 m-Drehbandkolonne unterworfen. Man erhielt 37,9 g Destillat, in dem das leichter siedende endo-Isomer **4** auf 80,6 % angereichert worden war sowie 40,8 g Destillationsrückstand, in dem das exo-Isomer **3** auf 95,1 % angereichert vorlag. Durch erneute Destillation des Destillats bzw. fortgesetzte Destillation des Destillationsrückstandes an einer 1 m-Drehbandkolonne wurde das endo-Isomer **4** in 98,3%iger Reinheit bzw. das exo-Isomer **3** in 99,6%iger Reinheit erhalten.

exo-Isomer **3**:

IR (Film): $\nu$ = 3329, 2937, 1478, 1451, 1078, 1034, 994 $cm^{-1}$.
$^1$H-NMR (300 MHz, $CDCl_3$): $\delta$ = 0.8-1.05 (m, 4 H), 1.09-1.32 (m, 3H), 1.51-1.67 (m, 2H), 1.72-1.90 (m, 4H), 1.94 ("s", 2H), 2.05 (br. s, 1H, O<u>H</u>), 3.33-3.43 ppm (m, 2H, C<u>H</u>$_2$OH).
$^{13}$C-NMR (125.7 MHz, $CDCl_3$): $\delta$ = 27.4 (t), 29.1 (t), 32.0 (t), 32.4 (t), 33.1 (t), 40.6 (d), 42.4 (d), 44.4 (d), 47.8 (d), 48.5 (d), 66.5 ppm (t).
MS: m/z (%) = 166 (0.4, $M^+$), 148 (12), 135 (100), 107 (19), 93 (16), 91 (16), 81 (13), 80 (18), 79 (37), 78 (11), 77 (14), 67 (47), 66 (10), 41 (18), 39 (14), 31 (15).

endo-Isomer **4:**

IR (Film): $\nu$ = 3316, 2958, 1475, 1452, 1070, 1056, 1035, 1014, 991 $cm^{-1}$.
$^1$H-NMR (300 MHz, $CDCl_3$): $\delta$ = 0.50-0.56 (m, 1H), 0.86-1.05 (m, 3H), 1.07-1.25 (m, 1H), 1.40-1.46 (m, 1H), 1.58-1.74 (m, 3H), 1.77-1.94 (m, 3H), 1.96-2.11 (m, 3H), 2.22 (br. s, 1H, O<u>H</u>), 3.48-3.60 (m, 2H, C<u>H</u>$_2$OH).
$^{13}$C-NMR (125,7 MHz, $CDCl_3$): $\delta$ = 27.1 (t), 32.2 (t), 32.7 (t), 32.8 (t), 33.7 (t), 40.2 (d), 41.1 (d), 41.9 (d), 42.1 (d), 48.4 (d), 64.2 ppm (t).
MS: m/z (%) = 166 (0.8, $M^+$), 148 (25), 135 (85), 133 (31), 120 (48), 119 (92), 107 (47), 106 (34), 105 (25), 95 (35), 94 (31), 93 (34), 91 (48), 81 (34), 80 (52), 79 (100), 78 (20), 77 (30), 67 (72), 66 (23), 41 (28), 39 (22), 31 (21).

Beispiel 3

Herstellung von 8-exo/endo-Formyl-2,6-exo-tricyclo [5.2.1.$0^{2,6}$]decan (**1/2**) mit einem erhöhten Anteil an 8-exo-Isomerem **1** (ausgehend von TCD-Keton-A)

Equilibrierung des 8-exo/endo-Aldehydgemisches **D (1/2**) aus Beispiel 1

5 g Aldehyd **D** aus Beispiel 1 (exo/endo = 58:40) wurden in einer Mischung aus 1 g 50 %iger Natronlauge und 25 g Methanol gelöst und 30 min unter Rückfluß erhitzt. Nach Abkühlung wurde mit 1 g Eisessig angesäuert und das Methanol im Vorvakuum abdestilliert. Der Rückstand wurde mit 25 g $H_2O$ versetzt und mit Diethylether extrahiert. Der etherische Extrakt wurde mit ges. $NaHCO_3$-Lösung und 5 %iger NaCl-Lösung neutralgewaschen, über $Na_2SO_4$ getrocknet und im 20 mm-Vakuum vom Lösungsmittel befreit. Nach Kugelrohrdestillation des Rückstandes (4.95 g) im 2 mm-Vakuum erhielt man 4.85 g Produkt als farbloses Öl.
Brechungsindex: $n_D^{20}$ = 1.5021 Dichte: $D_4^{20}$ = 1.0365
Nach Reduktion einer analytischen Probe mit $LiAlH_4$ analog Beispiel 2 wurde per Gaschromatographie ein exo/endo-Verhältnis von 84:16 ermittelt.

Darstellung des Aldehydgemisches **1/2** aus TCD-Keton A (**A**) durch modifizierte Glycidester-Synthese

Eine Lösung von 150 g (1 mol) TCD-Keton A (**A**), 165,4 g (1.35mol) Chloressigsäureethylester und 0,5 g Phenothiazin in 100 ml abs. Pyridin wurde unter Rühren und Kühlen innerhalb von 1h bei 15 °C portionsweise mit 81 g (1.5 mol) Natriummethylat versetzt. Nach Zusatz von 74 g tert-Butylmethylether wurde das Reaktionsgemisch weitere 4 h bei 15°C gerührt, dann unter Kühlen bei 15 °C mit einer Lösung von 125 g (1.56mol) 50%iger Natronlauge in 297 g Methanol versetzt und über Nacht ohne weitere Kühlung weitergerührt. Dann wurde mit 468 g (3.9mol) 50 %iger Essigsäure angesäuert und langsam zum Sieden erhitzt, wobei anfangs eine starke $CO_2$-Entwicklung auftritt. Nach zweistündigem Refluxieren wurden über eine 20cm-Vigreux-Kolonne 350 g Lösungsmittel abdestilliert. Anschließend wurde das erhaltene Reaktionsgemisch mit 600g 5%iger NaCl-Lösung verdünnnt und 2 mal mit je 150 ml Hexan extrahiert. Der Hexan-Extrakt wurde mit $NaHCO_3$-Lösung und Wasser neutralgewaschen und schließlich im 20 mm-Vakuum vom Lösungsmittel befreit. Man erhielt 155,2 g Rohaldehyd, der nach Gaschromatogramm noch 15% Keton **A** enthielt und ein exo/endo-Verhältnis von 4:1 aufweist (bestimmt per Gaschromatographie nach Reduktion einer analytischen Probe mit $LiAlH_4$ analog Beispiel 2).

Zur Abtrennung von Keton **A** aus dem Aldehydgemisch **1/2** wurden zu einer Lösung von 104 g $NaHSO_3$ in 2 l $H_2O$ unter Rühren in 30 min bei Raumtemperatur 155 g Rohaldehyd getropft. Die sich dabei bildende Suspension wurde eine weitere Stunde bei Raumtemperatur gerührt, bevor 2 mal mit je 150 ml Hexan extrahiert wurde. Dann wurde die wäßrige Phase mit 10 %iger Natronlauge alkalisiert, und das resultierende Zweiphasensystem 2 mal mit 250 ml Hexan extrahiert. Der Hexan-Extrakt wurde mit 5 %iger NaCl-Lösung neutralgewaschen und schließlich im 20 mm-Vakuum vom Lösungsmittel befreit. Das verbliebene Rohprodukt (117 g) wurde über eine 20 cm Vigreux-Kolonne destilliert. Man erhielt 101,6 g Aldehydgemisch **1/2** (62 % d.Th.) im Siedebereich 84 - 86 °C / 1,5 mm als farbloses Öl. GC-Reinheit: 97.1 %.

Brechungsindex: $n_D^{20}$ = 1,5020 Dichte: $D_4^{20}$ = 1,0366

Nach Reduktion einer analytischen Probe mit $LiAlH_4$ wurde per Gaschromatographie ein exo/endo-Verhältnis von 80:20 ermittelt.

Beispiel 4

Oxidation des Alkohols **3** aus Beispiel 2 (nach Trennung von Alkohol **4**)

Eine gut gerührte Suspension von 11,3 g (30 mmol) Pyridiniumdichromat in 28 ml abs. $CH_2Cl_2$ wurde in einer Stickstoffatmosphäre bei 0 °C mit einer Lösung von 3,32 g (20 mmol) Alkohol **3** in 12 ml abs. $CH_2Cl_2$ versetzt und anschließend ohne weitere Kühlung 9 h gerührt. Nach Verdünnung mit 150 ml Diethylether wurde die übersthende Lösung vom schwarzen Rückstand abdekantiert, und der Rückstand noch dreimal mit je 30 ml Diethylether gewaschen. Die vereinigten organischen Lösungen wurden über Kieselgel filtriert, mit 5 %iger Salzsäure gewaschen, mit $NaHCO_3$-Lösung und Wasser neutralgewaschen und schließlich im 20 mm-Vakuum vom Lösungsmittel befreit. Nach Kugelrohrdestillation des verbliebenen Rohproduktes (3,19 g) im 2 mm-Vakuum erhielt man 2,87 g Aldehyd **1** als farbloses Öl.
GC-Reinheit: 99.5 %.
IR (Film): $\nu$ = 2704, 1719 $cm^{-1}$ (Aldehyd).
$^1$H-NMR (300 MHz, $CDCl_3$): $\delta$ = 0.85 - 1.05 (m, 3H), 1.12-1.33 (m, 3H), 1.61-1.69 (m, 1H), 1.81-1.97 (m, 5H), 2.04-2.09 (m, 1H), 2.22-2.28 (m, 1H), 2.32 (br. "s", 1H), 9.66 ppm (d, J = 1.5 Hz, 1H, C$\overline{H}$O).
$^{13}$C-NMR (75 MHz, $CDCl_3$): $\delta$ = 27.3 (t), 29.5 (t), 30.1 (t), 31.9 (t), 32.1 (t), 40.4 (d), 42.2 (d), $\overline{4}$8.10 (d), 48.13 (d), 54.3 (d), 203.2 ppm (d).
MS: m/z (%) = 164 (4, $M^+$), 135 (61), 108 (32), 107 (100), 106 (21), 93 (27), 91 (20)), 79 (58), 77 (20), 67 (88), 41 (28), 39 (21).

Oxidation des Alkohols **4** aus Beispiel 2 (nach Trennung von Alkohol **3**)

Alkohol **4** wurde analog - wie unter a) beschrieben - in Aldehyd **2** überführt. GC-Reinheit: 98.1 %.
IR (Film): $\nu$ = 2710, 1719 $cm^{-1}$ (Aldehyd).
$^1$H-NMR (300 MHz, $CDCl_3$): $\delta$ = 0.85-1.04 (m, 2H), 1.07-1.22 (m, 2H), 1.50-1.66 (m, 4H), 1.74-1.96 (m, 4H), 2.04-2.08 (m, 1H), 2.48-2.51 (m, 1H), 2.65-2.72 (m, 1H)), 9.79 ppm (d, J = 1 Hz, 1H, C$\overline{H}$O).
$^{13}$C-NMR (75 MHz, $CDCl_3$): $\delta$ = 26.9 (t), 28.7 (t), 32.3 (t), 32.6 (t), 33.8 (t), 41.7 (d), 4$\overline{2}$.7 (d), 43.0 (d), 48.1 (d), 53.3 (d), 205.0 ppm (d).
MS: m/z (%) = 164 (3, $M^+$), 120 (100), 118 (16), 107 (22), 92 (17), 91 (31), 79 (44), 77 (16), 67 (28), 41

(20), 39 (16).

Beispiel 5

Herstellung von 8-exo/endo-Formyl-2,6-exo-tricyclo [5.2.1.$0^{2,6}$]decan (**1**/**2**) aus 8-Formyl-2,6-exo-tricyclo [5.2.1.$0^{2,6}$]decen-3(4) (**6**) durch Hydrierung

Eine Lösung von 370 g (2.28 mol) Aldehydgemisch **6** (erhalten durch regioselektive Hydroformylierung von **5**) in 185 g Methanol wurde mit 5 g $Na_2CO_3$ und 1 g Palladium auf Aktivkohle (5 % Pd) versetzt und 4 h in einer Wasserstoffatmosphäre bei 20 bar und 45 - 55 °C gerührt ($H_2$-Verbrauch: 102 % d.Th.). Nach Filtrieren und Einengen wurde das Hydriergut in 100 g Hexan aufgenommen, mit $H_2O$ neutralgewaschen und erneut eingeengt. Das verbliebene Rohprodukt (375 g) wurde über eine 1 m-Vigreux-Kolonne destilliert. Man erhielt 327,4 g Aldehydgemisch **1**/**2** (87.4 % d.Th.) im Siedebereich 84 - 86 °C/1,5 mm als farbloses Öl. GC-Reinheit: 98.5 %.

Nach Reduktion einer analytischen Probe mit $LiAlH_4$ wurde per Gaschromatographie ein exo/endo-Verhältnis von 81:19 ermittelt.

Beispiel 6

Herstellung von 8-exo/endo-Formyl-2,6-exo-tri-cyclo [5.2.1.$0^{2,6}$]decan (**1**/**2**) aus 2,6-exo-Tricyclo[5.2.1.$0^{2,6}$] decen-3 (**7**) durch Hydroformylierung

Eine Lösung von 402 g (3 mol) Olefin **7** in 400 g Toluol wurde mit einer Lösung von 75 mg dimerem Rhodium(II)-2-ethylhexanoat in 20 ml Toluol versetzt und in einer Synthesegasatmosphäre (Molverhältnis $CO/H_2$ = 1:1) 3 h bei 100 °C und 180 - 200 bar gerührt (Synthesegasverbrauch: 96.5 % d.Th.). Nach Abkühlen auf 30 °C wurde das Reaktionsgut destillativ bei 130 mbar vom Lösungsmittel und bei 2 mbar vom Katalysator getrennt. Das verbliebene Rohprodukt (486 g) wurde - wie in Beispiel 5 beschrieben - feindestilliert. Man erhielt 440,8 g Aldehydgemisch **1**/**2** (89.6 % d.Th.) als farbloses Öl. GC-Reinheit: 99.2 %. Nach Reduktion einer analytischen Probe mit $LiAlH_4$ wurde per Gaschromatographie ein exo/endo-Verhältnis von 11:1 ermittelt.

Beispiel 7

Bestimmung der Geruchsstärke von Aldehydgemischen **1**/**2**

Zur Geruchsbewertung wurden Lösungen der Aldehydgemische **1**/**2** hergestellt, die nach verschiedenen Verfahren in unterschiedlichen Isomerenverhältnissen erhalten worden. Als Lösungsmittel diente Isopropylmyristat. In Verdünnungsreihen wurde jeweils eine 1 %ige Lösung der Testprobe im Verhältnis 1:10 mit Isopropylmyristat weiter verdünnt.

Ein aus Parfümeuren bestehendes Testpanel ermittelte die Verdünnung, bei der keine Geruchswahrnehmung mehr möglich war. Die Konzentration für den so bestimmten Geruchsschwellenwert ist durch Unterstreichen markiert.

Hierbei stellte sich heraus, daß 8-exo-angereicherte Aldehydgemische **1**/**2** bei erheblich höherer Verdünnung noch wahrnehmbar sind, d. h. eine höhere Geruchsintensität aufweisen als das nach bekanntem Verfahren (Beispiel 1) hergestellte Produkt.

| **1**/**2** (58:40) gemäß Bsp.1 | **1**/**2** (84:16) gemäß Bsp.3a | **1**/**2** (81:19) gemäß Bsp.5 | **1**/**2** (80:20) gemäß Bsp.3b |
|---|---|---|---|
| 1 % | 1 % | 1 % | 1 % |
| 0.1 % | 0.1 % | 0.1 % | 0.1 % |
| 0.01 % | 0.01 % | 0.01 % | 0.01 % |
| 0.001 % | 0.001 % | 0.001 % | 0.001 % |
| 0.0001 % | 0.0001 % | 0.0001 % | 0.0001 % |
| 0.00001 % | 0.00001 % | 0.00001 % | 0.00001 % |
| 0.000001 % | 0.000001 % | 0.000001 % | 0.000001 % |

Beispiel 8

Parfüm-Akkord

| | a | b | c |
|---|---|---|---|
| Dihydromyrcenol | 150 | 150 | 150 |
| Patchouliöl Indonesisch | 100 | 100 | 100 |
| Timberol* | 100 | 100 | 100 |
| Aldehyd **D** (gemäß Beispiel 1) | - | 5 | - |
| Formyltricyclodecap **1/2** (gemäß Beispiel 3a) | - | - | 5 |
| Dipropylenglycol | 645 | 645 | 645 |

* DRAGOCO-Produkt

Die Mischung a ist ein Parfumakkord mit holzigen, blumigen und süß-krautigen Aspekten. Durch Zugabe von 0.5 % Aldehyd **D** (Mischung b) wird der Akkord harmonischer unter gleichzeitiger Betonung grün-blumiger Aspekte. Bei alternativer Zugabe von 0.5 % Formyltricyclodecan **1/2** (Mischung c) erhält man einen Akkord, der stärker ist als (b) und zugleich frischer blumig riecht.

Beispiel 9

Parfümöl vom Chypre-Typ

| | a | b |
|---|---|---|
| Basilikumöl | 1,5 | 1,5 |
| Galbanumöl | 4,5 | 4,5 |
| Dimethylbenzylcarbinylacetat | 7,5 | 7,5 |
| Jasmin Base* | 30,0 | 30,0 |
| Mandarinenöl | 30,0 | 30,0 |
| Hexylzimtaldehyd | 45,0 | 45,0 |
| Lignofix©* | 45,0 | 45,0 |
| Moschusketon | 45,0 | 45,0 |
| Vetiverylacetat | 60,0 | 60,0 |
| Linalylacetat | 90,0 | 90,0 |
| cis-3-Hexenylsalicylat | 90,0 | 90,0 |
| Phenylethylalkohol | 90,0 | 90,0 |
| Methylionone, gamma | 75,0 | 75,0 |
| Benzylsalicylat | 109,0 | 109,0 |
| Bergamottöl | 120,0 | 120,0 |
| Lyral©** | 150,0 | 150,0 |
| Formyltricyclodecan **1/2** (gemäß Beispiel 3a) | - | 7,5 |

* DRAGOCO-Produkt
** IFF-Produkt

Die Mischung a besitzt einen ausgewogenen Duft vom Chypre-Typ mit Agrumen-Aspekten. Durch Zugabe von 0.75 % Formyltricyclodecan **1/2** (Mischung b) verschiebt sich der Dufteindruck in sehr gewünschter Weise in frisch-blumige Richtung mit Betonung unterschwelliger grün-würziger Note.

Beispiel 10

Parfümöl für Waschmittel

| | a | b |
|---|---|---|
| Sandranol[c] * | 5 | 5 |
| Styrolylacetat | 10 | 10 |
| Aldehyd C14 | 10 | 10 |
| Rosenoxid-D* 10 %ig | 10 | 10 |
| Cyclogalbanat* | 15 | 15 |
| Isodamascon* 1 %ig | 20 | 20 |
| Buccoxime[c]* 1 %ig | 20 | 20 |
| Citronellol | 20 | 20 |
| Hexylsalicylat | 20 | 20 |
| Ketofix* | 20 | 20 |
| Vetikolacetat* | 25 | 25 |
| Geraniol | 30 | 30 |
| Lilial[c] *** | 50 | 50 |
| Phenylethylalkohol | 50 | 50 |
| Greenylacetat* | 50 | 50 |
| Benzylacetat | 60 | 60 |
| Greenylpropionat* | 60 | 60 |
| Lignofix[c] * | 60 | 60 |
| Benzylsalicylat | 70 | 70 |
| Methylionone, gamma | 70 | 70 |
| Galaxolide[c] ** 50%ig | 100 | 100 |
| Hexylzimtaldehyd alpha | 100 | 100 |
| Lyral[c] ** | 105 | 105 |
| Dipropylenglycol | 15 | 15 |
| Formyltricyclodecan **1/2** (gemäß Beispiel 3a) | - | 5 |

* DRAGOCO- Produkt
** IFF-Produkt
*** Givaudan-Produkt

Die Mischung a zeigt eine ausgewogene süß-blumige Note, die sich gut für die Parfümierung von Waschmitteln und Wäscheweichspülern eignet. Die durch Hinzufügen von 0.5 % Formyltricyclodecan **1/2** erhaltene Mischung b vermittelt einen "weicheren" Geruchseindruck. Der blumig-pflegende Eindruck ist in beiden Fällen auch nach einem Waschvorgang auf den gewaschenen Textilien wahrnehmbar, wobei in Mischung b frisch-blumige Noten betont werden.

## Patentansprüche

## Patentansprüche für folgende Vertragsstaaten : CH, LI, DE, FR, GB, NL

1. 8-exo-Formyl-2,6-exo-tricyclo $[5.2.1.0^{2,6}]$decan (**1**)

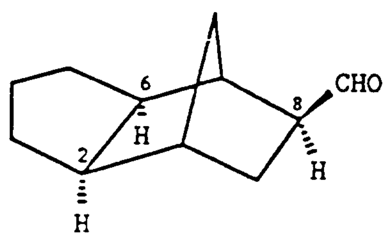

1 (d, l)

2. Verfahren zur Herstellung von 8-exo-Formyl-2,6-exo-tri-cyclo$[5.2.1.0^{2,6}]$decan (**1**), dadurch gekennzeichnet, daß in bekannter Weise erhaltenes 8-exo/endo-Formyl-2,6-exo-tricyclo$[5.2.1.0^{2,6}]$decan (exo/endo-Verhältnis = 1,45:1) unter Basenkatalyse, vorzugsweise durch Einwirkung von wässerigmethanolischer Natronlauge bei Siedetemperatur, in ein Aldehydgemisch mit erhöhtem C-8-exo-Anteil (exo/endo = 5,25:1) umgewandelt wird.

3. Verfahren zur Herstellung von 8-exo-Formyl-2,6-exo-tricyclo$[5.2.1.0^{2,6}]$decan (**1**), dadurch gekennzeichnet, daß 8-Formyl-2,6-exo-tricyclo $[5.2.1.0^{2,6}]$decen-3(4) in an sich bekannter Weise einer katalytischen Hydrierung, vorzugsweise unter Verwendung von Palladiumkohle, und wahlweise einer nachfolgenden Equilibrierung gemäß Anspruch 2 unterzogen wird.

4. Verfahren zur Herstellung von 8-exo-Formyl-2,6-exo-tricyclo$[5.2.1.0^{2,6}]$decan (**1**), dadurch gekennzeichnet, daß 2,6-exo- Tricyclo$[5.2.1.0^{2,6}]$ decen-8 in an sich bekannter Weise hydroformyliert und wahlweise gemäß Anspruch 2 equilibriert wird.

5. Verfahren zur Herstellung von reinem 8-exo-Formyl-2,6-exo- tricyclo$[5.2.1.0^{2,6}]$decan (**1**), dadurch gekennzeichnet, daß Verfahrensprodukte gemäß Ansprüchen 2, 3 oder 4 in an sich bekannter Weise zum korrespondierenden Alkoholgemisch reduziert werden, aus dem durch chromatographische oder destillative Methoden das 8-exo-konfigurierte Methylol isoliert und in ebenfalls bekannter Weise zu **1** oxidiert wird.

6. Verwendung von 8-exo-Formyl-2,6-exo-tricyclo $[5.2.1.0^{2,6}]$decan (**1**) als Riechstoff bzw.Bestandteil von Riechstoffmischungen und für kosmetische und technische Anwendungen.

7. Verwendung von 8-exo-angereicherten Gemischen von 8-exo/endo-Formyl-2,6-exo-tricyclo$[5.2.1.0^{2,6}]$-decan mit einem exo-Isomerenanteil von mindestens 70 % und einem endo-Isomerenanteil von höchstens 30 %, als geruchsgebendes Agens bzw. als Bestandteil von Parfümölkompositionen, für kosmetische und technische Anwendungen.

**Patentansprüche für folgenden Vertragsstaat : ES**

1. Verfahren zur Herstellung von 8-exo-Formyl-2,6-exo-tri-cyclo[5.2.1.$0^{2,6}$]decan (**1**)

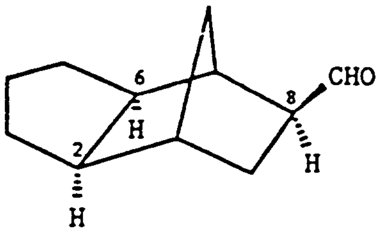

1 (d, l)

dadurch gekennzeichnet, daß in bekannter Weise erhaltenes 8-exo/endo-Formyl-2,6-exo-tricyclo-[5.2.1.$0^{2,6}$]decan (exo/endo-Verhältnis = 1,45:1) unter Basenkatalyse, vorzugsweise durch Einwirkung von wässerigmethanolischer Natronlauge bei Siedetemperatur, in ein Aldehydgemisch mit erhöhtem C-8-exo-Anteil (exo/endo = 5,25:1) umgewandelt wird.

2. Verfahren zur Herstellung von 8-exo-Formyl-2,6-exo-tricyclo[5.2.1.$0^{2,6}$]decan (**1**), dadurch gekennzeichnet, daß 8-Formyl-2,6-exo-tricyclo [5.2.1.$0^{2,6}$]decen-3(4) in an sich bekannter Weise einer katalytischen Hydrierung, vorzugsweise unter Verwendung von Palladiumkohle, und wahlweise einer nachfolgenden Equilibrierung gemäß Anspruch 1 unterzogen wird.

3. Verfahren zur Herstellung von 8-exo-Formyl-2,6-exo-tricyclo[5.2.1.$0^{2,6}$]decan (**1**), dadurch gekennzeichnet, daß 2,6-exo- Tricyclo[5.2.1.$0^{2,6}$] decen-8 in an sich bekannter Weise hydroformyliert und wahlweise gemäß Anspruch 1 equilibriert wird.

4. Verfahren zur Herstellung von reinem 8-exo-Formyl-2,6-exo- tricyclo[5.2.1.$0^{2,6}$]decan (**1**), dadurch gekennzeichnet, daß Verfahrensprodukte gemäß Ansprüchen 1, 2 oder 3 in an sich bekannter Weise zum korrespondierenden Alkoholgemisch reduziert werden, aus dem durch chromatographische oder destillative Methoden das 8-exo-konfigurierte Methylol isoliert und in ebenfalls bekannter Weise zu **1** oxidiert wird.

5. Verwendung von 8-exo-Formyl-2,6-exo-tricyclo [5.2.1.$0^{2,6}$]decan (**1**) als Riechstoff bzw.Bestandteil von Riechstoffmischungen und für kosmetische und technische Anwendungen.

6. Verwendung von 8-exo-angereicherten Gemischen von 8-exo/endo-Formyl-2,6-exo-tricyclo[5.2.1.$0^{2,6}$]-decan mit einem exo-Isomerenanteil von mindestens 70 % und einem endo-Isomerenanteil von höchstens 30 %, als geruchsgebendes Agens bzw. als Bestandteil von Parfümölkompositionen, für kosmetische und technische Anwendungen.

## Claims

**Claims for the following Contracting States : CH, DE, FR, GB, NL, LI**

1. 8-exo-formyl-2,6-exo-tricyclo [5.2.1.$0^{2,6}$]decane (**1**)

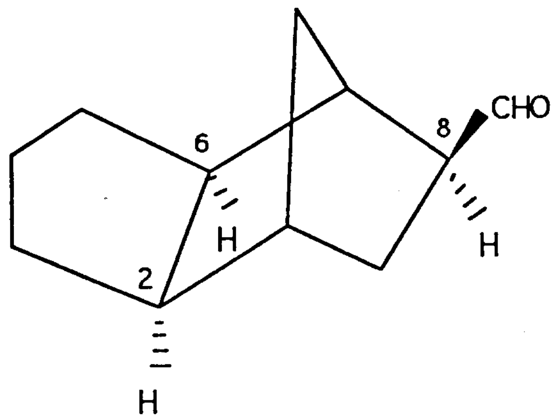

1 (d, l)

2. A process for the production of 8-exo-formyl-2,6-exo-tricyclo [5.2.1.$0^{2,6}$]decane (**1**), characterised in that 8-exo/endo-formyl-2,6-exo-tricyclo[5.2.1.$0^{2,6}$]decane (exo/endo ratio = 1.45:1) obtained in a known manner is transformed into an aldehyde mixture having an increased C-8-exo-ratio (exo/endo - 5.25:1) using base catalysis, preferably by the effect of an aqueous methanolic solution of caustic soda at boiling temperature.

3. A process for the production of 8-exo-formyl-2,6-exo-tricyclo[5.2.1.$0^{2,6}$]decane (**1**), characterised in that 8-formyl-2,6-exo-tricyclo[5.2.1.$0^{2,6}$]decene-3(4) is subjected to a known catalytic hydrogenation, preferably using palladium carbon, which may be followed by an equilibration according to claim 2.

4. A process for the production of 8-exo-formyl-2,6-exo-tricyclo[5.2.1.$0^{2,6}$]decane (**1**) characterised in that 2,6-exo-tricyclo[5.2.1.$0^{2,6}$]decene-8 is hydroformylated in a known manner, which may be equilibrated according to claim 2.

5. A process for the production of pure 8-exo-formyl-2,6-exo-tricyclo[5.2.1.$0^{2,6}$]decane (**1**) characterised in that the process products in accordance with claims 2, 3 or 4 are reduced in a known manner to the corresponding alcohol mixture from which the 8-exo-configuration methylol is isolated by chromatographic or distillation methods and that there is an oxidation to **1** in an also known manner.

6. The use of 8-exo-formyl-2,6-exo-tricyclo[5.2.1.$0^{2,6}$] decane (**1**) as a scent or a component of scent mixtures and for cosmetic and technical uses.

7. The use of 8-exo-enriched mixtures of 8-exo/endo-formyl-2,6-exo-tricyclo[5.2.1.$0^{2,6}$]decane having an exo-isomer proportion of at least 70% and an endo-isomer proportion of at most 30% used as scent-imparting agent or as a component of perfume oil compositions, for cosmetic and technical uses.

**Claims for the following Contracting State : ES**

1. A process for the production of 8-exo-formyl-2,6-exo-tricyclo [5.2.1.0$^{2,6}$]decane (**1**)

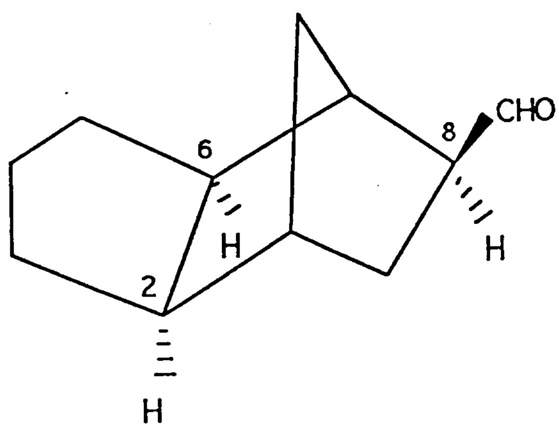

1 (d, l)

characterised in that 8-exo/endo-formyl-2,6-exo-tricyclo[5.2.1.0$^{2,6}$]decane (exo/endo ratio = 1.45:1) obtained in a known manner is transformed into an aldehyde mixture having an increased C-8-exo-ratio (exo/endo - 5.25:1) using base catalysis, preferably by the effect of an aqueous methanolic solution of caustic soda at boiling temperature.

2. A process for the production of 8-exo-formyl-2,6-exo-tricyclo[5.2.1.0$^{2,6}$]decane (**1**), characterised in that 8-formyl-2,6-exo-tricyclo[5.2.1.0$^{2,6}$]decene-3(4) is subjected to a known catalytic hydrogenation, preferably using palladium carbon, which may be followed by an equilibration according to claim 1.

3. A process for the production of 8-exo-formyl-2,6-exo-tricyclo[5.2.1.0$^{2,6}$]decane (**1**) characterised in that 2,6-exo-tricyclo[5.2.1.0$^{2,6}$]decene-8 is hydroformylated in a known manner, which may be equilibrated according to claim 1.

4. A process for the production of pure 8-exo-formyl-2,6-exo-tricyclo[5.2.1.0$^{2,6}$]decane (**1**) characterised in that the process products in accordance with claims 1, 2 or 3 are reduced in a known manner to the corresponding alcohol mixture from which the 8-exo-configuration methylol is isolated by chromatographic or distillation methods and that there is an oxidation to **1** in an also known manner.

5. The use of 8-exo-formyl-2,6-exo-tricyclo [5.2.1.0$^{2,6}$] decane (**1**) as a scent or a component of scent mixtures and for cosmetic and technical uses.

6. The use of 8-exo-enriched mixtures of 8-exo/endo-formyl-2,6-exo-tricyclo[5.2.1.0$^{2,6}$]decane haying an exo-isomer proportion of at least 70% and an endo-isomer proportion of at most 30% used as scent-imparting agent or as a component of perfume oil compositions, for cosmetic and technical uses.

**Revendications**

**Revendications pour les Etats contractants suivants : CH, DE, FR, GB, NL, LI**

1. 8-exo-Formyl-2,6-exo-tricyclo [5.2.1.$0^{2},^{6}$] décane (1)

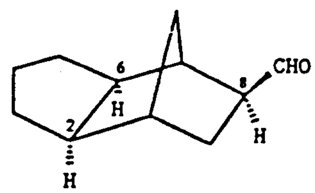

1 (d, 1)

2. Procédé de fabrication de 8-exo-Formyl-2,6-exo-tri-cyclo[5.2.1. $0^{2},^{6}$] décane (1) , caractérisé en ce que du 8-exo/endo-Formyl-2,6-exo -tricyclo[5.2.1.$0^{2},^{6}$] décane (rapport exo/endo = 1,45:1) obtenu d'une manière connue, est transformé sous catalyse basique, de préférence par l'action de soude caustique méthanolique aqueuse à la température d'ébullition, en un mélange d'aldéhydes avec une portion de C-8-exo (exo/endo = 5,25:1) augmentée.

3. Procédé de fabrication de 8-exo-Formyl-2,6-exo-tricyclo[5.2.1. $0^{2},^{6}$] décane (1), caractérisé en ce que du 8-Formyl-2,6-exo-tricyclo [5.2.1.$0^{2},^{6}$] décène-3(4) est soumis, d'une façon connue en soi, à une hydratation catalytique, de préférence en utilisant du charbon de palladium, et au besoin à un équilibrage successif suivant la revendication 2.

4. Procédé de fabrication de 8-exo-Formyl-2,6-exo-tricyclo[5.2.1. $0^{2},^{6}$] décane (1), caractérisé en ce que du 2,6-exo- tricyclo [5.2.1.$0^{2},^{6}$] décène-8 est, d'une façon connue en soi, hydroformylé et au besoin équilibré suivant la revendication 2.

5. Procédé de fabrication de 8-exo-Formyl-2,6-exo- tricyclo[5.2.1. $0^{2},^{6}$] décane (1) à l'état pur, caractérisé en ce que les produits du procédé suivant les revendications 2, 3 ou 4 sont, de façon connue en soi, réduits en mélange d'alcools correspondants, d'où est isolé, par méthode de chromatographie ou de distillation, le méthylol configuré 8-exo, puis est oxydé à 1, d'une manière éventuellement connue.

6. Utilisation de 8-exo-Formyl-2,6-exo-tricyclo [5.2.1.$0^{2},^{6}$] décane (1) comme matière odorante en composant de mélanges de matières odorantes et pour des applications cosmétiques et techniques.

7. Utilisation de mélanges enrichis en 8-exo de 8-exo/endo-Formyl-2,6-exo-tricyclo[5.2.1.$0^{2},^{6}$] décane avec une proportion d'isomère exo d'au moins 70 % et une proportion d'isomère endo d'au plus 30 %, code agent chimique odoriférant comme composant de compositions d'essences de parfum, pour des applications cosmétiques et techniques.

**Revendications pour l'Etat contractant suivant : ES**

1. Procédé pour la fabrication de 8-exo-formyl-2,6-exo-tricyclo (5.2.1.$0^{2.6)}$-decan (1)

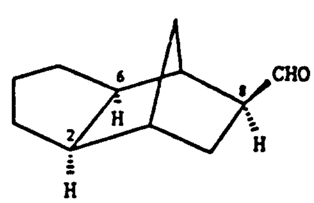

1 (d, 1)

caractérisé en ce que du 8-exo/endo-formyl-2,6-exo-tricyclo(5.2.1.02.6)decan (rapport exo/endo =

1,45:1) obtenu d'une manière connue est transformé sous catalyse basique, de préférence par action de lessive de soude caustique méthanolique aqueuse à la température d'ébullition, en un mélange d'aldéhydes avec portion en C-8-exo (exo/endo = 5,25:1) augmentée.

**2.** Procédé de fabrication de 8-exo-formyl-2,6-exo-tricyclo (5.2.1.02.6)decan (1), caractérisé en ce que du 8-formyl-2,6-exi-tricyclo(5.2.1.02.6)decen-3(4) est soumis, d'une manière connue en soi, à une hydratation catalytique, de préférence en utilisant du charbon palladeux, et facultativement à un équilibrage subséquent suivant la revendication 1.

**3.** Procédé de fabrication de 8-exo-formyl-2,6-exo-tricyclo (5.2.1.02.6)decan (1), caractérisé en ce que du 2,6-exo-tricyclo (5.2.1.02.6)decen-8 est, d'une manière connue en soi, hydroformylé et facultativement équilibré suivant la revendication 1.

**4.** Procédé de fabrication de 8-exo-formyl-2,6-exo-tricyclo (5.2.1.02.6)decan (1) à l'état pur, caractérisé en ce que les produits du procédé suivant les revendications 1, 2 ou 3 sont, d'une manière connue en soi, réduits en mélange d'alcools correspondants, d'où est isolé, par méthode chromatographique ou de distillation, le méthylol configuré en 8-exo, puis est oxydé à 1, d'une manière éventuellement connue.

**5.** Utilisation de 8-exo-formyl-2,6-exo-tricyclo(5.2.1.02.6) (1) comme matière odorante ou composant de mélanges de matières odorantes et pour applications cosmétiques et techniques.

**6.** Utilisation de mélanges de enrichis en 8-exo de 8-exo/endo-formyl-2,6-exo-tricyclo(5.2.1.02.6)decan avec une portion isomère exo d'au moins 70 % et une portion isomère endo d'au plus 30 %, comme agent chimique odoriférant ou comme composant de compositions d'essences de parfum, pour applications cosmétiques et techniques.